(19) Europäisches Patentamt
European Patent Office
Office européen des brevets

(11) EP 3 897 729 B1

(12) **EUROPEAN PATENT SPECIFICATION**

(45) Date of publication and mention of the grant of the patent:
**13.03.2024 Bulletin 2024/11**

(21) Application number: **19836895.3**

(22) Date of filing: **17.12.2019**

(51) International Patent Classification (IPC):
***A61K 47/38*** *(2006.01)* ***A61K 9/48*** *(2006.01)*
***C08L 1/28*** *(2006.01)* ***C08B 11/02*** *(2006.01)*

(52) Cooperative Patent Classification (CPC):
**A61K 47/38; A61K 9/0065; A61K 9/4808; A61K 9/4866; C08B 11/02; C08L 1/28**

(86) International application number:
**PCT/US2019/066704**

(87) International publication number:
**WO 2020/131780 (25.06.2020 Gazette 2020/26)**

(54) **A SUSTAINED RELEASE COMPOSITION COMPRISING A METHYLCELLULOSE**

ZUSAMMENSETZUNG MIT EINER METHYLCELLULOSE MIT VERZÖGERTER FREISETZUNG

COMPOSITION À LIBÉRATION PROLONGÉE COMPRENANT UNE MÉTHYLCELLULOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2018 EP 18213440**

(43) Date of publication of application:
**27.10.2021 Bulletin 2021/43**

(73) Proprietor: **DDP Speciality Electronics Materials US, Inc.**
**Collegeville, PA 19426 (US)**

(72) Inventor: **PETERMANN, Oliver**
**29699 Bomlitz (DE)**

(74) Representative: **International N&H EMEA**
**Parallelvej 16**
**2800 Kongens Lyngby (DK)**

(56) References cited:
**WO-A1-2013/059064**

- **MITCHELL K ET AL: "The influence of substitution type on the performance of methylcellulose and hydroxypropylmethycellulose in gels and matrices", INTERNATIONAL JOURNAL OF PHARMACEUTICS, ELSEVIER, NL, vol. 100, no. 1-3, 8 November 1993 (1993-11-08), pages 143-154, XP025554113, ISSN: 0378-5173, DOI: 10.1016/0378-5173(93)90085-T [retrieved on 1993-11-08]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD

The present invention relates to novel sustained release compositions comprising a physiologically active ingredient and a methylcellulose.

INTRODUCTION

Sustained release dosage forms have found wide application in a variety of technology areas such as in personal care and agricultural applications, water treatment and in particular pharmaceutical applications. Sustained release dosage forms are designed to release a finite quantity of an active ingredient into an aqueous environment over an extended period of time. Sustained release pharmaceutical dosage forms are desirable because they provide a method of delivering a long-lasting dose in a single application without overdosing. Known sustained release pharmaceutical dosage forms contain a drug or a vitamin whose release is controlled by a polymeric matrix which, for instance, may comprise one or more water-soluble cellulose ethers. Water-soluble cellulose ethers hydrate on the surface of a tablet to form a gel layer. A fast formation of the gel layer is important to prevent wetting of the interior and disintegration of the tablet core. Once the gel layer is formed, it controls the penetration of additional water into the tablet. As the outer layer fully hydrates and dissolves, an inner layer must replace it and be sufficiently cohesive and continuous to retard the influx of water and control drug diffusion.

A commonly used cellulose ether for providing sustained release of an active ingredient from an oral dosage form is hydroxypropyl methylcellulose (HPMC). For instance, US 4,734,285 discloses that the release of an active ingredient can be prolonged by employing a fine particle sized HPMC as an excipient in a solid tablet. HPMC is used in commercial oral pharmaceutical formulations as a component of a polymeric matrix providing sustained release of a drug usually at a concentration of 30% to 60% by weight of the oral dosage form.

Mitchell K et al., 1993, International Journal of Pharmaceutics 100(1-3): 143-154, investigated the influence of substitution type on the performance of methylcellulose and hydroxypropylmethylcellulose in gels and matrices containing a model drug.

It is a well-known problem in the pharmaceutical art that some patients, especially children or the elderly, or patients with dysphagia, find it difficult to swallow conventional oral dosage forms such as capsules or tablets. In particular, this is the case if the drug administered in the dosage form is a highly dosed drug which, when the drug is formulated with pharmaceutical excipients in the typical amounts included in commercial dosage forms, either makes each dosage form very large or requires the dose to be divided among two or more dosage forms that have to be swallowed at the same time.

It would therefore be desirable to develop an oral dosage form where a drug is formulated with a reduced amount of excipient(s) to permit a reduction in the overall size of the dosage form and improve the swallowability without compromising the sustained release properties thereof.

SUMMARY OF THE INVENTION

It has surprisingly been found that when a methylcellulose which is capable of forming a stable hydrogel at body temperature in an aqueous environment is used as an excipient in admixture with a physiologically active ingredient, it can provide sustained release of the active ingredient in the stomach of an individual ingesting the dosage form. Without wishing to be limited by theory, it is assumed that when the dosage form is prepared using an amount of liquid diluent which is below the amount of solids in the dosage form, i.e. 85% or less by weight of the solids, a hydrogel can form from the methylcellulose because it is slowly hydrated in an aqueous environment such as the stomach, whereas if the amount of liquid diluent used to prepare the dosage form is above the amount of solids, the methylcellulose will be diluted in the stomach before a hydrogel can form.

Accordingly, the present invention relates to a sustained release composition for oral administration comprising a physiologically active ingredient mixed with a methylcellulose, wherein

the methylcellulose has anhydroglucose units joined by 1-4 linkages and wherein hydroxy groups of anhydroglucose units are substituted with methyl groups such that the s23/s26 is 0.27 or less,
wherein s23 is the molar fraction of anhydroglucose units wherein only the two hydroxy groups in the 2- and 3-positions of the anhydroglucose unit are substituted with methyl groups and
wherein s26 is the molar fraction of anhydroglucose units wherein only the two hydroxy groups in the 2- and 6-positions of the anhydroglucose unit are substituted with methyl groups,
the composition further comprising a liquid diluent in a weight ratio of liquid diluent to active ingredient up to 0.85:1,

wherein the weight ratio of liquid diluent to solids is of 0.1:1 to 0.85:1.

A methylcellulose capable of forming a hydrogel at body temperature (in the following referred to as low temperature gelling, or LTG, methylcellulose) has been disclosed in the literature as an excipient in pharmaceutical and nutraceutical formulations. Thus, WO 2015/009796 discloses a liquid composition for mucosal application comprising LTG methylcellulose, a physiologically active agent and a liquid diluent. The methylcellulose forms a gel after application to a mucosa, e.g. the nasal cavity, and the active agent is delivered transmucosally from the gel.

EP 2997098 B1 discloses methylcellulose with a s23/s26 ratio of 0.36 or less which is used as a coating on non-starch, water-soluble polysaccharides to improve their palantability when they are used as dietary fibers, making them less slimy and tacky on ingestion.

US 2017/0260414 discloses a methylcellulose which gels at a temperature of 13-25oC as a 15% by weight aqueous solution. The methylcellulose is applied on solid dosage forms as a taste-masking coating.

US 9,700,630 B2 discloses a low molecular weight methylcellulose which has a gelation temperature in the range of 35-40°C. The methylcellulose is included in a liquid sustained release composition intended for parenteral drug delivery. The methylcellulose forms a gel upon injection into a tissue and is slowly degraded so as to release the drug.

The use of methylcellulose as a controlled release matrix material is disclosed in K.S. Aithal et al., Indian J. Dent. Res. 1, Apr-Sep 1990, 174-181. Chewable tablets containing 2% by weight of NaF, 30% by weight of methylcellulose, 63% by weight of lactose and 5% by weight of starch powder. The authors report that about 80% of the NaF is released within 20 minutes from this formulation, while the release of NaF from tablets containing either more or less methylcellulose is about 15 minutes. There is no indication that methylcellulose may be used as an excipient providing sustained release over a period of several hours.

The present inventor is not aware of any literature that discloses the use of an LTG methylcellulose as an excipient of a polymeric matrix to provide sustained release of an active ingredient from a solid dosage form upon oral administration.

Thus, in another aspect, the invention relates to the use of a methylcellulose composed of anhydroglucose units joined by 1-4 linkages, wherein hydroxy groups of anhydroglucose units are substituted with methyl groups such that the s23/s26 is 0.27 or less,

wherein s23 is the molar fraction of anhydroglucose units wherein only the two hydroxy groups in the 2- and 3-positions of the anhydroglucose unit are substituted with methyl groups and
wherein s26 is the molar fraction of anhydroglucose units wherein only the two hydroxy groups in the 2- and 6-positions of the anhydroglucose unit are substituted with methyl groups,
as an excipient of a polymeric matrix of a composition providing sustained release of an active ingredient from an oral solid dosage form, which composition further comprising a liquid diluent in a weight ratio of liquid diluent to active ingredient up to 0.85:1, wherein the weight ratio of liquid diluent to solids is of 0.1:1 to 0.85:1.

In a further aspect, the invention relates to a process for preparing a sustained release composition for oral administration comprising a physiologically active ingredient mixed with a methylcellulose, the process comprising

(a) providing a solution of methylcellulose in a liquid diluent,
(b) mixing the active ingredient in powder or crystalline form and optionally one or more solid excipients with the solution of methylcellulose in a weight ratio of liquid diluent to solids of 0.1:1 to 0.85:1, and
(c) optionally drying the mixture of step (b) to a liquid content of less than 10% by weight of the mixture,

said methylcellulose being composed of anhydroglucose units joined by 1-4 linkages, wherein hydroxy groups of anhydroglucose units are substituted with methyl groups such that the s23/s26 is 0.27 or less,
wherein s23 is the molar fraction of anhydroglucose units wherein only the two hydroxy groups in the 2- and 3-positions of the anhydroglucose unit are substituted with methyl groups and
wherein s26 is the molar fraction of anhydroglucose units wherein only the two hydroxy groups in the 2- and 6-positions of the anhydroglucose unit are substituted with methyl groups.

BRIEF DESCRIPTION OF THE DRAWINGS

Fig. 1 is a graph showing the release over time of acetaminophen (APAP) from a composition of the invention containing a 2% solution of LTG methylcellulose when a gelatin capsule containing the composition is immersed in 900 ml of 0.1 N HCl pH 1.1.

Fig. 2 is a graph showing the release over time of acetaminophen (APAP) from a composition of the invention containing a 4% solution of LTG methylcellulose when a gelatin capsule containing the composition is immersed in 900 ml of 0.1 N HCl pH 1.1.

Fig. 3 is a graph showing the release over time of acetaminophen (APAP) from a composition of the invention containing a 2% solution of LTG methylcellulose dried overnight, when a gelatin capsule containing the dried composition is immersed in 900 ml of 0.1 N HCl pH 1.1.

Fig. 4 is a graph showing the release over time of acetaminophen (APAP) from a composition of the invention containing a 2% solution of LTG methylcellulose dried overnight (shown as -♦-) and from a composition containing a 2% solution of Methocel A4M methylcellulose dried overnight (shown as - ▪ -).

Fig. 5 is a graph showing the release over time of acetaminophen (APAP) from a composition of the invention containing a 4% solution of LTG methylcellulose and $CaCO_3$ dried overnight, when a gelatin capsule containing the dried composition is immersed in 900 ml of 0.1 N HCl pH 1.1.

Fig. 6 is a graph showing the release of metroprolol tartrate from dry compressed matrix tablets containing either 30% by weight LTG methylcellulose (shown as - x -), 30% by weight of HPMC K100 M (shown as - ♦ -) or 90% by weight of a filler (shown as - □ -).

DESCRIPTION OF EMBODIMENTS

In the present invention, the liquid diluent is preferably an aqueous liquid containing 50-100% water and may for instance be selected from purified water or water containing a surfactant acting as a defoaming aid during preparation of the composition. The weight ratio of liquid diluent to active ingredient is preferably in the range of 0:1 to 0.75:1, 0:1 to 0.70:1, 0:1 to 0.65:1, 0:1 to 0.60:1, 0:1 to 0.55:1, 0:1 to 0.50:1, 0:1 to 0.45:1 or 0:1 to 0.40:1. The weight ratio "0:1" is intended to indicate that the composition is dried, that is, contains substantially no added liquid.

In the present invention, a specific methylcellulose that can form a hydrogel in an aqueous environment such as the stomach is an essential component of the composition to provide sustained release of the active ingredient on oral administration of the composition even when the methylcellulose is present in a very low amount relative to the active ingredient. The methylcellulose has anhydroglucose units joined by 1-4 linkages. Each anhydroglucose unit contains hydroxyl groups at the 2, 3, and 6 positions. Partial or complete substitution of these hydroxyls creates cellulose derivatives. For example, treatment of cellulosic fibers with caustic solution, followed by a methylating agent, yields cellulose ethers substituted with one or more methoxy groups. If not further substituted with other alkyls, this cellulose derivative is known as methylcellulose.

An essential feature of the specific methylcellulose used in the composition of the present invention is the position of the methyl groups. The composition of the invention comprises a methylcellulose wherein hydroxy groups of anhydroglucose units are substituted with methyl groups such that s23/s26 is 0.27 or less, preferably 0.26 or less, more preferably 0.24 or less or even 0.22 or less. In this embodiment of the invention s23/s26 of the methylcellulose typically is 0.08 or more, 0.10 or more, 0.12 or more, 0.14 or more, 0.16 or more, or 0.18 or more.

In the ratio s23/s26, s23 is the molar fraction of anhydroglucose units wherein only the two hydroxy groups in the 2- and 3-positions of the anhydroglucose unit are substituted with methyl groups and s26 is the molar fraction of anhydroglucose units wherein only the two hydroxy groups in the 2- and 6-positions of the anhydroglucose unit are substituted with methyl groups. For determining the s23, the term "the molar fraction of anhydroglucose units wherein only the two hydroxy groups in the 2- and 3-positions of the anhydroglucose unit are substituted with methyl groups" means that the two hydroxy groups in the 2- and 3-positions are substituted with methyl groups and the 6-positions are unsubstituted hydroxy groups. For determining the s26, the term "the molar fraction of anhydroglucose units wherein only the two hydroxy groups in the 2- and 6-positions of the anhydroglucose unit are substituted with methyl groups" means that the two hydroxy groups in the 2- and 6-positions are substituted with methyl groups and the 3-positions are unsubstituted hydroxy groups.

Formula I below illustrates the numbering of the hydroxy groups in anhydroglucose units.

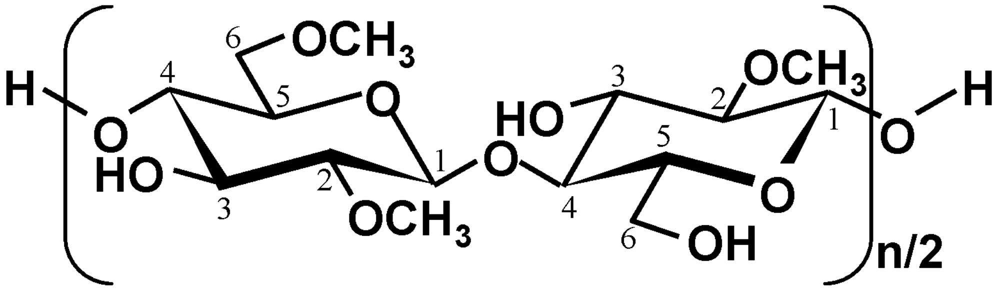

Formula I

In one embodiment of the invention hydroxy groups of anhydroglucose units are substituted with methyl groups such that the s23/s26 of the methylcellulose is 0.27 or less, preferably 0.26 or less, more preferably 0.25 or less, more preferably 0.24 or less or even 0.22 or less. In this embodiment of the invention s23/s26 of the methylcellulose typically is 0.08 or more, 0.10 or more, 0.12 or more, 0.14 or more, 0.16 or more, or 0.18 or more.

The methylcellulose included in the present composition preferably has a DS(methyl) of from 1.55 to 2.25, more preferably from 1.65 to 2.20, and most preferably from 1.70 to 2.10. The degree of the methyl substitution, DS(methyl), also designated as DS(methoxyl), of a methylcellulose is the average number of OH groups substituted with methyl groups per anhydroglucose unit.

The determination of the % methoxyl in methylcellulose is carried out according to the United States Pharmacopeia (USP 34). The values obtained are % methoxyl. These are subsequently converted into degree of substitution (DS) for methyl substituents. Residual amounts of salt have been taken into account in the conversion.

The viscosity of the methylcellulose included in the present composition is generally at least 2.4 mPa•s, preferably at least 3 mPa•s, and most preferably at least 10 mPa•s, when measured as a 2 wt. % aqueous solution at 5 °C at a shear rate of 10 $s^{-1}$. The viscosity of the methylcellulose is preferably up to 10,000 mPa•s, more preferably up to 5000 mPa•s, and most preferably up to 2000 mPa•s, when measured as indicated above.

In an aqueous environment, the methylcellulose included in the present composition is capable of gelling at body temperature (about 35-37°C), forming stable hydrogels in an aqueous environment even at very low concentrations of methylcellulose resulting in a satisfactory sustained release performance. The term "stable hydrogels", when used in this context is intended to mean hydrogels that retain their shape and are not completely dissolved or significantly eroded after immersion in 0.1 N HCl, pH 1.1, for 4 hours at 37°C. The methylcellulose is referred to as low temperature gelling (LTG) herein. The gelation temperature may for instance be determined as described in WO 2015/009796.

Methods of making methylcellulose are described in more detail in the Examples. Generally, cellulose pulp is treated with a caustic, for example alkali metal hydroxide. Preferably, about 1.5 to about 3.0 mol NaOH per mol anhydroglucose units in the cellulose is used. Uniform swelling and alkali distribution in the pulp is optionally controlled by mixing and agitation. The rate of addition of aqueous alkaline hydroxide is governed by the ability to cool the reactor during the exothermic alkalization reaction. In one embodiment, an organic solvent such as dimethyl ether is added to the reactor as a diluent and coolant. Likewise, the headspace of the reactor is optionally purged with an inert gas (such as nitrogen) to minimize unwanted reactions with oxygen and molecular weight losses of the methylcellulose. In one embodiment, the temperature is maintained at or below 45°C.

A methylating agent such as methylene chloride is also added by conventional means to the cellulose pulp either before or after or concurrently with the caustic, generally in an amount of 2.0 to 3.5 mol methylating agent per mol anhydroglucose units in the cellulose. Preferably, the methylating agent is added after the caustic. Once the cellulose has been contacted with caustic and methylating agent, the reaction temperature is increased to about 75°C and reacted at this temperature for about half an hour.

In a preferred embodiment, a staged addition is used, i.e. a second amount of caustic is added to the mixture over at least 30 minutes, preferably at least 45 minutes, while maintaining the temperature at 20 to 70°C. Preferably 2 to 4 mol caustic per mol of anhydroglucose units in the cellulose is used. A staged second amount of methylating agent is added to the mixture either before, after or concurrently with the caustic, generally in an amount of 2 to 4.5 mol methylating agent per mol of anhydroglucose units in the cellulose. Preferably, the second amount of methylating agent is added prior to the second amount of caustic.

The methylcellulose is washed to remove salt and other reaction by-products. Any solvent in which salt is soluble may be employed, but water is preferred. The methylcellulose may be washed in the reactor, but is preferably washed in a separate washer located downstream of the reactor. Before or after washing, the methylcellulose may be stripped by exposure to steam to reduce residual organic content. The cellulose ether may subsequently be subjected to a partial depolymerizing process. Partial depolymerizing processes are known in the art and described in e.g. EP 1141029, EP 210917, EP 1423433 and US 4316982. Alternatively, partial depolymerization can be achieved during the

production of the cellulose ether, for example by the presence of oxygen or an oxidizing agent.

The methylcellulose is dried to a reduced moisture and volatile content of preferably 0.5 to 10.0% by weight of water and more preferably 0.8 to 5.0% by weight of water and volatiles based on the weight of methylcellulose. The reduced moisture and volatiles content enables the methylcellulose to be milled into particulate form. The methylcellulose is milled to particulates of a desired size. If desired, drying and milling may be carried out simultaneously.

The LTG methylcellulose described above is useful as an excipient for a sustained release dosage form which means that it has the function to regulate the release of an active ingredient from the dosage form over an extended period of time. The term "sustained release" is used herein synonymously with the term "controlled release". Sustained release is an approach by which active ingredients such as physiologically active compounds are made available at a rate and duration designed to accomplish an intended effect. The LTG methylcellulose is useful for forming all or part of a polymeric matrix in which the active ingredient is embedded. The polymeric matrix may additionally comprise one or more other polymers capable of providing sustained release of an active ingredient from a dosage form. The LTG methylcellulose typically constitutes at least 50% by weight, preferably 60-100%, more preferably 70-100%, even more preferably 80-100%, and most preferably 90-100% by weight of the polymeric matrix. When one or more other polymers are included in the polymeric matrix, they may be selected from cellulose ethers such as hydroxypropyl methylcellulose (HPMC), hydroxyethyl methylcellulose, hydroxypropyl cellulose, methylcellulose with s23/s26 between 0.27 and 0.36 or between 0.38 and 0.42 or carboxymethylcellulose, or another polysaccharide such as sodium alginate or calcium alginate. However, it is generally preferred that the LTG methylcellulose constitutes 100% by weight of the polymeric matrix.

The LTG methylcellulose may be included in sustained release dosage forms, in particular dosage forms intended for oral administration of drugs or other physiologically active ingredients and release thereof into the gastrointestinal tract so as to control the absorption rate of the active ingredient to achieve a desired blood plasma profile. The combined amount of LTG methylcellulose and active ingredient(s) in the dosage form is preferably at least 50%, more preferably at least 70% and most preferably at least 90% by dry weight of the dosage form, and up to 100%, preferably up to 98% and most preferably up to 95% by dry weight of the dosage form.

The dosage form is designed to provide a constant or nearly constant level of the active ingredient in plasma with reduced fluctuation via a slow, continuous release of the active ingredient over an extended period of time such as a period of between 4 and 30 hours, preferably between 8 and 24 hours to release all or almost all of the active ingredient from the dosage form.

It has been found that sustained release dosage forms such as tablets and capsules wherein the polymer matrix is formed partially or completely from LTG methylcellulose remain intact over an extended time period such as at least 4 hours, preferably at least 6 hours and under optimized conditions at least 8 hours. Without wanting to be bound by theory, it is believed that the LTG methylcellulose is hydrated to form a strong swollen layer on the outer surface of the dosage form upon contact with an aqueous liquid at body temperature. The strong swollen layer minimizes the release of the active ingredient caused by erosion of the dosage form. Since the tablets or capsule contents do not disintegrate (i.e. do not fall apart to any significant degree), the release of the active ingredient is controlled by the slow diffusion from the swollen layer that has been formed by hydration of the methylcellulose on the outer surface of the dosage form. A strong swollen layer reduces the penetration of water into the sustained release dosage form, which delays the release of the active ingredient, particularly a water-soluble active ingredient, into an aqueous environment due to a reduced amount of water in the zone of the dosage form into which water diffuses and dissolves the active ingredient.

While the concentration of LTG methylcellulose may vary within a wide range in the composition and may for instance be included in a concentration of up to 60% by weight of the active ingredient, it has surprisingly been found that essentially the same rate of release of the active ingredient can be achieved with a much lower amount of LTG methylcellulose as all or part of the polymeric matrix. Thus, it has been found that essentially the same rate of release of the active ingredient can be achieved as with commercial sustained release dosage forms that typically contain about 30% by weight of HPMC when the LTG methylcellulose is included in the dosage form as the sole matrix polymer in a concentration of 0.1-10%, preferably 0.2-5.0%, more preferably 0.5-4.0%, more preferably 0.75-2.0% and still more preferably 0.8-1.5% by dry weight of the active ingredient. In one embodiment, the LTG methylcellulose is included as the sole matrix polymer in the dosage form in a concentration of about 1% by dry weight of the active ingredient. The resulting sustained release dosage form, such as tablet or capsule, is smaller in size and therefore easier to ingest. It has furthermore been found that a satisfactory release rate may be obtained without adding any other excipients to the dosage form though a surfactant may optionally be added during the manufacturing process as a defoaming agent.

In an embodiment, the composition comprises an additive which on ingestion reacts with gastric fluid to generate a gas such as $CO_2$. The developing gas is trapped in the hydrogel which, as a result, floats to the surface of the gastric contents resulting in prolonged gastric retention time. The prolonged gastric retention time improves the bioavailability of the active ingredient, increases the duration of release and improves the solubility of active ingredients that are not readily soluble in the high pH environment of the intestine. Examples of additives which generate gas in contact with gastric fluid are alkali metal or alkaline earth metal carbonates such as $CaCO_3$ or $Na_2CO_3$. The concentration of the

additive may be in the range of 1-5% by weight, preferably 1.5-3% by weight, such as 2% by weight of the composition.

The present composition may suitably be prepared by providing a solution of LTG methylcellulose in a liquid diluent such as water in step (a) of the present process, optionally adding a surfactant to the solution as a processing aid. The active ingredient in powder or crystalline form and optionally one or more solid excipients may then be mixed with the LTG methylcellulose solution in step (b) of the present process. The amount of liquid diluent mixed with solids in step (b) is at least 10% by weight, preferably at least 20% by weight, more preferably at least 30% by weight and most preferably at least 40% by weight of the solids, and up to 85% by weight, preferably up to 75% by weight, more preferably up to 70% by weight, even more preferably up to 60% by weight, and most preferably up to 50% by weight of the solids. The term "solids" is understood to mean the combination of active ingredient(s), LTG methylcellulose and optionally one or more other solid excipients in the composition.

Addition of a surfactant helps to distribute a low level of liquid diluent homogenously and produce a smooth highly viscous semi-solid paste, possibly due to defoaming and emulsification. The surfactant may be selected from conventional defoaming agents selected from the group consisting of anionic surfactants with anionic functional groups such as sulfates, sulfonates, phosphates and carboxylates such as alkyl sulfates, e.g. ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate; cationic surfactants with cationic functional groups such as cetrimonium bromide (CTAB), cetylpyridinium chloride (CPC), benzalkonium chloride (BAC), benzethonium chloride (BZT), dimethyldioctadecylammonium chloride, dioctadecyldimethylammonium bromide (DODAB); zwitterionic surfactants such as cocamidopropyl betaine; and nonionic surfactants such as siloxane surfactants like modified polydimethylsiloxane-based defoamer, ethoxylates, fatty acid esters of glycerol, sorbitol and sucrose.

The concentration of surfactant may be in the range of 0.1-1.5% by weight of the composition.

In one embodiment of the invention, the composition comprising LTG methylcellulose admixed with the active ingredient is in the form of a dry powder. The dry powder may be prepared in step (c) of the present process by drying the mixture of the methylcellulose solution and active ingredient at a temperature of 40-100°C until the mixture has a water content of less than 10% by weight, preferably less than 5% by weight, more preferably less than 3% by weight, in particular less than 2% by weight, such as less than 1% by weight, followed by milling or grinding the mixture to granules of a desired particle size in a manner known in the art. The dry powder will typically contain granules comprising the active ingredient partially or completely encased by methylcellulose which facilitates sustained release of the active ingredient as discussed above.

In one embodiment, the invention relates to a unit dosage form comprising the present composition. The unit dosage form is intended for oral administration and may be in the form of a tablet comprising compressed granules of the dried composition. Alternatively, the unit dosage form may be in the form of a tablet or pellet prepared by extruding the semi-solid paste prepared as described above and cutting the extruded mass into pieces of an appropriate size followed by drying. The tablet may optionally comprise one or more other excipients, though preferably the LTG methylcellulose is the only excipient included in the dosage form, except that a surfactant and/or an additive capable of reacting with gastric fluid to generate a gas may optionally be included as indicated above. The unit dosage form may also be a capsule including the dried composition, preferably in the form of dry granules containing the mixture of LTG methylcellulose and active ingredient. The unit dosage form may also be in the form of a syringe or pouch pre-filled with the wet mixture: this dosage form may more readily be administered to young children.

The unit dosage form contains one or more physiologically active ingredients, preferably one or more drugs, one or more diagnostic agents, or one or more physiologically active ingredients which are useful for cosmetic or nutritional purposes. The term "drug" denotes a compound having beneficial prophylactic and/or therapeutic properties when administered to an individual, typically a mammal, especially a human individual. The dosage form is believed to be particularly suited for administering highly dosed drugs, i.e. drugs administered in unit doses of 500 mg or more, as it is possible to provide a unit dose that includes the requisite amount of the active ingredient in a size that makes it easier to ingest. Examples of highly dosed drugs are metformin, metformin hydrochloride, acetaminophen (paracetamol) or acetylsalicylic acid. Thus, each unit dosage form may typically include 500-1000 mg of the active ingredient.

Some embodiments of the invention will now be described in detail in the following Examples.

Unless otherwise mentioned, all parts and percentages are by weight. In the Examples the following test procedures are used.

<u>Production of Methylcellulose</u>

Methylcellulose were produced according to the following procedure. Finely ground wood cellulose pulp was loaded into a jacketed, agitated reactor. The reactor was evacuated and purged with nitrogen to remove oxygen, and then evacuated again. The reaction is carried out in two stages. In the first stage, a 50% by weight aqueous solution of sodium hydroxide was sprayed onto the cellulose until the level reached 1.8 mol of sodium hydroxide per mol of anhydroglucose units of the cellulose, and then the temperature was adjusted to 40 °C. After stirring the mixture of aqueous

sodium hydroxide solution and cellulose for about 20 minutes at 40 °C, 1.5 mol of dimethyl ether and 2.3 mol of methyl chloride per mol of anhydroglucose units were added to the reactor. The contents of the reactor were then heated in 60 min to 80 °C. After having reached 80 °C, the first stage reaction was allowed to proceed for 5 min. Then the reaction was cooled down to 65 °C in 20 min.

The second stage of the reaction was started by addition of methyl chloride in an amount of 3.4 molar equivalents of methyl chloride per mol of anhydroglucose unit. The addition time for methyl chloride was 20 min. Then a 50% by weight aqueous solution of sodium hydroxide at an amount of 2.9 mol of sodium hydroxide per mol of anhydroglucose units was added over a period of 45 min. The rate of addition was 0.064 mol of sodium hydroxide per mol of anhydroglucose units per minute. After the second-stage addition was completed the contents of the reactor were heated up to 80 °C in 20 min and then kept at a temperature of 80 °C for 120 min.

After the reaction, the reactor was vented and cooled down to about 50 °C. The contents of the reactor were removed and transferred to a tank containing hot water. The crude methylcellulose was then neutralized with formic acid and washed chloride free with hot water (assessed by $AgNO_3$ flocculation test), cooled to room temperature and dried at 55 °C in an air-swept drier, and subsequently ground. The methylcellulose had a DS(methyl) of 1.88 (30.9 wt.% methoxyl), a mol fraction (26-Me) of 0.3276 ± 0.0039, a mol fraction (23-Me) of 0.0642 ± 0.0060, an s23/s26 of 0.20 ± 0.02, and a steady-shear-flow viscosity $\eta$(5 °C, 10 $s^{-1}$, 2 wt.% MC) of 5500 mPa•s. The properties of the methylcellulose were measured as described below.

<u>Determination of s23/s26 of Methylcellulose</u>

The approach to measure the ether substituents in methylcellulose is generally known. See for example the approach described in principle for Ethyl Hydroxyethyl Cellulose in Carbohydrate Research, 176 (1988) 137-144, Elsevier Science Publishers B.V., Amsterdam, DISTRIBUTION OF SUBSTITUENTS IN O-ETHYL-O-(2-HYDROXYETHYL)CELLULOSE by Bengt Lindberg, Ulf Lindquist, and Olle Stenberg.

Specifically, determination of s23/s26 was conducted as follows:

10-12 mg of the methylcellulose were dissolved in 4.0 mL of dry analytical-grade dimethyl sulfoxide (DMSO) (Merck, Darmstadt, Germany, stored over 0.3nm molecular sieve beads) at about 90 °C with stirring and then cooled to room temperature. The solution was stirred at room temperature over night to ensure complete solubilization/dissolution. The entire perethylation including the solubilization of the methylcellulose was performed using a dry nitrogen atmosphere in a 4 mL screw cap vial. After solubilization, the dissolved methylcellulose was transferred to a 22-mL screw-cap vial to begin the perethylation process. Powdered sodium hydroxide (freshly pestled, analytical grade, Merck, Darmstadt, Germany) and ethyl iodide (for synthesis, stabilized with silver, Merck-Schuchardt, Hohenbrunn, Germany) were introduced in a thirty-fold molar excess relative to the level of anhydroglucose units in the methylcellulose, and the mixture was vigorously stirred under nitrogen in the dark for three days at ambient temperature. The perethylation was repeated with addition of the threefold amount of the reagents sodium hydroxide and ethyl iodide compared to the first reagent addition, and stirring at room temperature was continued for an additional two days. Optionally, the reaction mixture could be diluted with up to 1.5 mL DMSO to ensure good mixing during the course of the reaction. Next, five mL of 5 % aqueous sodium thiosulfate solution was poured into the reaction mixture, and the mixture was then extracted three times with 4 mL of dichloromethane. The combined extracts were washed three times with 2 mL of water. The organic phase was dried with anhydrous sodium sulfate (about1 g). After filtration, the solvent was removed with a gentle stream of nitrogen, and the sample was stored at 4 °C until needed.

Hydrolysis of about 5 mg of the perethylated samples was performed under nitrogen in a 2-mL screw-cap vial with 1 mL of 90 % aqueous formic acid under stirring at 100 °C for 1 hour. The acid was removed in a stream of nitrogen at 35-40 °C and the hydrolysis was repeated with 1 mL of 2M aqueous trifluoroacetic acid for 3 hours at 120 °C in an inert nitrogen atmosphere with stirring. After completion, the acid was removed to dryness in a stream of nitrogen at ambient temperature using ca. 1 mL of toluene for co-distillation.

The residues of the hydrolysis were reduced with 0.5 mL of 0.5-M sodium borodeuteride in 2N aqueous ammonia solution (freshly prepared) for 3 hours at room temperature with stirring. The excess reagent was destroyed by dropwise addition of about 200 $\mu$L of concentrated acetic acid. The resulting solution is evaporated to dryness in a stream of nitrogen at about 35-40 °C and subsequently dried in vacuum for 15 min at room temperature. The viscous residue was dissolved in 0.5 mL of 15 % acetic acid in methanol and evaporated to dryness at room temperature. This was done five times and repeated four additional times with pure methanol. After the final evaporation, the sample was dried in vacuum overnight at room temperature.

The residue of the reduction was acetylated with 600 $\mu$L of acetic anhydride and 150 $\mu$L of pyridine for 3 hrs at 90 °C. After cooling, the sample vial was filled with toluene and evaporated to dryness in a stream of nitrogen at room temperature. The residue was dissolved in 4 mL of dichloromethane and poured into 2 mL of water and extracted with 2 mL of dichloromethane. The extraction was repeated three times. The combined extracts were washed three times with 4 mL of water and dried with anhydrous sodium sulfate. The dried dichloromethane extract was subsequently

submitted to GC analysis. Depending on the sensitivity of the GC system, a further dilution of the extract could be necessary.

Gas-liquid (GLC) chromatographic analyses were performed with Agilent 6890N type of gas chromatographs (Agilent Technologies GmbH, 71034 Boeblingen, Germany) equipped with Agilent J&W capillary columns (30 m, 0.25-mm ID, 0.25-μm phase layer thickness) operated with 1.5-bar helium carrier gas. The gas chromatograph was programmed with a temperature profile that held constant at 60 °C for 1 min, heated up at a rate of 20 °C / min to 200 °C, heated further up with a rate of 4 °C / min to 250 °C, and heated further up with a rate of 20 °C / min to 310 °C where it was held constant for another 10 min. The injector temperature was set to 280 °C and the temperature of the flame ionization detector (FID) was set to 300 °C. Exactly 1μL of each sample was injected in the splitless mode at 0.5-min valve time. Data were acquired and processed with a LabSystems Atlas work station.

Quantitative monomer composition data were obtained from the peak areas measured by GLC with FID detection. Molar responses of the monomers were calculated in line with the effective carbon number (ECN) concept but modified as described in the table below. The effective carbon number (ECN) concept has been described by Ackman (R.G. Ackman, J. Gas Chromatogr., 2 (1964) 173-179 and R.F. Addison, R.G. Ackman, J. Gas Chromatogr., 6 (1968) 135-138) and applied to the quantitative analysis of partially alkylated alditol acetates by Sweet et. Al (D.P. Sweet, R.H. Shapiro, P. Albersheim, Carbohyd. Res., 40 (1975) 217-225).

<u>ECN increments used for ECN calculations:</u>

| Type of carbon atom | ECN increment |
|---|---|
| hydrocarbon | 100 |
| primary alcohol | 55 |
| secondary alcohol | 45 |

In order to correct for the different molar responses of the monomers, the peak areas were multiplied by molar response factors MRFmonomer which are defined as the response relative to the 2,3,6-Me monomer. The 2,3,6-Me monomer were chosen as reference since it was present in all samples analyzed in the determination of s23 / s26.

$$\text{MRFmonomer} = \text{ECN2,3,6-Me} / \text{ECNmonomer}$$

The mol fractions of the monomers were calculated by dividing the corrected peak areas by the total corrected peak area according to the following formulas:

(1) s23 is the sum of the molar fractions of anhydroglucose units which meet the following condition [the two hydroxy groups in the 2- and 3-positions of the anhydroglucose unit are substituted with methyl groups, and the 6-position is not substituted (= 23-Me)]; and

(2) s26 is the sum of the molar fractions of anhydroglucose units which meet the following condition [the two hydroxy groups in the 2- and 6-positions of the anhydroglucose unit are substituted with methyl groups, and the 3-position is not substituted (= 26-Me)].

<u>Determination of the DS(methyl) a methylcellulose</u>

The determination of the % methoxyl in methylcellulose was carried out according to the United States Pharmacopeia (USP34). The values obtained were % methoxyl. These were subsequently converted into degree of substitution (DS) for methyl substituents. Residual amounts of salt were taken into account in the conversion.

<u>Production of a 2 % pure aqueous solution of the methylcellulose</u>

To obtain a 2 % aqueous solution of methylcellulose, 3 g of milled, ground, and dried methylcellulose (under consideration of the water content of the methylcellulose) were added to 147 g of tap water (temperature 20 - 25 °C) at room temperature while stirring with an overhead lab stirrer at 750 rpm with 3-wing (wing = 2 cm) blade stirrer. The solution was then cooled to about 1.5 °C. After the temperature of 1.5 °C was reached the solution was stirred for 180

min at 750 rpms. Prior to use or analysis, the solution was stirred for 15 min at 100 rpm in an ice bath.

Determination of the viscosity of methylcellulose

The steady-shear-flow viscosity η(5 °C, 10 $s^{-1}$, 2 wt.% MC) of an aqueous 2-wt.% methylcellulose solution was measured at 5 °C at a shear rate of 10 $s^{-1}$ with an Anton Paar Physica MCR 501 rheometer and cup and bob fixtures (CC-27).

**Example 1: Release of Acetaminophen from gelatin capsules comprising LTG Methylcellulose**

A 2% by weight aqueous solution of LTG methylcellulose was prepared and a modified polydimethylsiloxane-based defoamer (available from BASF under the tradename Foamstar SI 2210) was added to the solution. 10.0 g of acetaminophen (abbreviated herein to APAP) was intimately mixed with 5.0 g of the solution of LTG methylcellulose until a white homogenous and highly viscous paste was obtained. The content of Foamstar SI2210 in the paste was 0.0885 g. The mixture was filled into a syringe and injected into gelatin capsules (size 000) which were subsequently closed and sealed. Each capsule contained 2 mg/ml APAP. The filled capsules were immediately placed in 900 ml of 0.1N HCl pH 1.1 at 37°C and shaken at 150 rpm for 22 hours. Drug release was measured at a wavelength of 243 nm with a path length of 0.1 mm.

The release of APAP from the capsule is shown in Fig. 1 from which it appears that about 75% of the APAP was released from each capsule after 24 hours.

Visual inspection showed that even after 24 hours of immersion in 0.1N HCl a capsule of hydrogel with a solid core of active ingredient was clearly visible.

A similar experiment was made with capsules loaded with a mixture of APAP mixed with a 4% aqueous solution of LTG methylcellulose. It appears from Fig. 2 that about 75% of the APAP was released from the capsules over 24 hours in a zero order fashion.

Gelatin capsules containing APAP mixed with a 2% solution of LTG methylcellulose were placed in USP phosphate buffer pH 7.4 under similar conditions as described above to mimic conditions in the intestine/colon. Visual inspection showed that even after 24 hours of immersion in phosphate buffer pH 7.4, a capsule of hydrogel with a solid core of active ingredient was clearly visible. It was concluded that the dissolution profile of the active ingredient from the capsule was similar when the capsule was immersed in phosphate buffer as in HCl pH 1.1, i.e. the composition of the invention can be used to obtain controlled release of an active ingredient both in the stomach and in the intestine.

**Example 2: Release of Acetaminophen from dried gelatin capsules containing LTG methylcellulose**

A 2% by weight aqueous solution of LTG methylcellulose was prepared and a modified polydimethylsiloxane-based defoamer (available from BASF under the tradename Foamstar SI 2210) was added to the solution. 10.0 g of APAP was intimately mixed with 5.0 g of the solution of LTG methylcellulose until a white homogenous and highly viscous paste was obtained. The content of Foamstar SI2210 in the paste was 0.0885 g. Gelatin capsules (size 000) were filled with about 1 g of the paste and subsequently closed. The capsules were then dried carefully overnight at 50°C. Each capsule contained about 1 g of APAP and 10 mg LTG methylcellulose. The dried capsules were placed in 900 ml of 0.1N HCl pH 1.1 at 37°C and shaken at 150 rpm for 22 hours. Drug release was measured at a wavelength of 243 nm with a path length of 0.1 mm.

The release of APAP from the capsules is shown in Fig. 3 from which it appears that about 75% of the drug was released after 24 hours.

Visual inspection showed that even after 22 hours of immersion in 0.1N HCl pH 1.1 a capsule of hydrogel with a small solid core of active ingredient was clearly visible. The release of the active ingredient thus appeared to be similar from a dry composition as from a wet composition.

**Example 3: Release of Acetaminophen from dried gelatin capsules containing LTG methylcellulose and A4M methylcellulose**

2% by weight aqueous solutions of LTG methylcellulose and Methocel A4M methylcellulose (available from DuPont) were prepared and a modified polydimethylsiloxane-based defoamer (available from BASF under the tradename Foamstar SI 2210) was added to each solution. A4M methylcellulose has a DS(methyl) of 1.82, an s23/s26 of 0.38-0.42 and a steady-shear-flow viscosity η (5°C, 10 $s^{-1}$, 2% by weight methylcellulose) of 4580 mPa.s. 10.0 g of APAP was intimately mixed with 5.0 g of the solution of LTG methylcellulose and A4M methylcellulose, respectively, until a white homogenous and highly viscous paste was obtained. The content of Foamstar SI2210 in each paste was 0.0885 g. Gelatin capsules (size 000) were filled with about 1 g of each paste and subsequently closed. The capsules were then

dried carefully overnight at 50°C. Each capsule contained about 1 g of APAP and 10 mg LTG methylcellulose or A4M methylcellulose. The dried capsules were placed in 900 ml of 0.1N HCl pH 1.1 at 37°C and shaken at 150 rpm for 22 hours. Drug release was measured at a wavelength of 243 nm with a path length of 0.1 mm.

The release of APAP from the capsules is shown in Fig. 4 from which it appears that about 75% of the drug was released after 24 hours from the capsules containing LTG methylcellulose as the sustained release polymeric matrix, whereas about 90% of the drug was released after 6 hours from the capsules containing A4M methylcellulose. A polymeric matrix composed of LTG methylcellulose therefore appears to be particularly suitable for oral dosage forms for once daily administration.

## Example 4: Release of Acetaminophen from dried gelatin capsules containing LTG methylcellulose and $CaCO_3$

A 4% by weight aqueous solution of LTG methylcellulose was prepared and 0.196 g of $CaCO_3$ was added to the solution. 6.373 g of APAP was intimately mixed with the solution until a homogenous and highly viscous paste was obtained. Gelatin capsules (size 000) were filled with about 1 g of the paste and subsequently closed. The capsules were then dried for 24 hours in an oven at 60°C.

The dried capsules were placed in 900 ml of 0.1N HCl pH 1.1 at 37°C and shaken at 150 rpm for 22 hours. Drug release was measured at a wavelength of 243 nm with a path length of 0.1 mm.

The release of APAP from the dried capsules is shown in Fig. 5 from which it appears that about 80% of the drug was released after 22 hours (shown as -•- in the figure).

## Example 5 (Comparative): Release of metroprolol tartrate from matrix tablets comprising LTG methylcellulose or HPMC K100M as the matrix polymer

Tablets were produced using dry powder blends of HPMC K100M (available from DuPont), LTG methylcellulose, metroprolol tartrate, dicalcium phosphate and magnesium stearate with different ratios according to the table below. Tablets were pressed with a manual IR tablet press (Perkin-Elmer, Norwalk, USA) with a compression force of 7.5t.

| Excipient A | % Excipient | wt of excipient (g) | Excipient B | % Excipient | wt of excipient (g) | Drug | % Drug | wt of drug (g) | Filler | % filler | wt of filler (g) | Lube | % Lube | wt of Lube (g) | Total wt (g) |
|---|---|---|---|---|---|---|---|---|---|---|---|---|---|---|---|
| Methocel K100M | 30 | 3 | LTG- MC | 0 | 0 | Met. Tart. | 10 | 1 | DCP | 59,5 | 5,95 | stearate | 0,5 | 0.05 | 10 |
| Methocel K100M | 0 | 0 | LTG- MC | 30 | 3 | Met. Tart. | 10 | 1 | DCP | 59,5 | 5,95 | stearate | 0,5 | 0.05 | 10 |
| Methocel K100M | 0 | 0 | LTG- MC | 0 | 0 | Met. Tart. | 10 | 1 | DCP | 89,5 | 8,95 | stearate | 0,5 | 0.05 | 10 |

The release of metroprolol tartrate is shown in Fig. 6 from which it appears that matrix tablets comprising LTG methylcellulose prepared by dry mixing of the components followed by compression into tablets released 100% of the active ingredient within 20 minutes (shown as - x -) as did matrix tablets prepared with 90% by weight of dicalcium phosphate (shown as - □ -), whereas matrix tablets comprising HPMC K100M prepared by dry mixing of the components followed by compression released about 90% of the active ingredient within 600 minutes (shown as - ♦ -).

## Claims

1. A sustained release composition for oral administration comprising a physiologically active ingredient mixed with a methylcellulose, wherein

   the methylcellulose has anhydroglucose units joined by 1-4 linkages and wherein hydroxy groups of anhydroglucose units are substituted with methyl groups such that the s23/s26 is 0.27 or less,
   wherein s23 is the molar fraction of anhydroglucose units wherein only the two hydroxy groups in the 2- and 3-positions of the anhydroglucose unit are substituted with methyl groups and
   wherein s26 is the molar fraction of anhydroglucose units wherein only the two hydroxy groups in the 2- and 6-positions of the anhydroglucose unit are substituted with methyl groups,
   the composition further comprising a liquid diluent in a weight ratio of liquid diluent to active ingredient up to 0.85:1, wherein the weight ratio of liquid diluent to solids is of 0.1:1 to 0.85:1.

2. The composition of claim 1, wherein the weight ratio of liquid diluent to active ingredient is up to 0.75:1, preferably up to 0.70:1, more preferably up to 0.65:1, to 0.60:1,to 0.55:1, to 0.50:1, to 0.45:1 or to 0.40:1.

3. The composition of claim 1 or 2, wherein the concentration of methylcellulose is 0.1-10.0%, preferably 0.2-5.0%, more preferably 0.5-4.0%, more preferably 0.75-2.0% and still more preferably 0.8-1.5%, such as 1%, by dry weight of the active ingredient.

4. The composition of any one of claims 1 to 3 comprising a methylcellulose wherein hydroxy groups of anhydroglucose units are substituted with methyl groups such that s23/s26 is 0.26 or less, preferably 0.25 or less, more preferably 0.24 or less, such as 0.22 or less.

5. The composition of any one of claims 1 to 4 further comprising a surfactant.

6. The composition of any one of claims 1 to 5, wherein the concentration of the surfactant is in the range of 0.1-1.5% by weight of the composition.

7. The composition of any one of claims 1 to 6 further comprising an additive capable of reacting with gastric fluid to generate a gas.

8. The composition of claim 7, wherein the additive is selected from alkali metal or alkaline earth metal carbonates such as $CaCO_3$ or $Na_2CO_3$.

9. A unit dosage form comprising a composition according to any one of claims 1-8.

10. A unit dosage form according to claim 9, wherein the active ingredient is selected from the group consisting of metformin, metformin hydrochloride, acetaminophen and acetylsalicylic acid.

11. Use of a methylcellulose composed of anhydroglucose units joined by 1-4 linkages, wherein hydroxy groups of anhydroglucose units are substituted with methyl groups such that the s23/s26 is 0.27 or less,

    wherein s23 is the molar fraction of anhydroglucose units wherein only the two hydroxy groups in the 2- and 3-positions of the anhydroglucose unit are substituted with methyl groups and
    wherein s26 is the molar fraction of anhydroglucose units wherein only the two hydroxy groups in the 2- and 6-positions of the anhydroglucose unit are substituted with methyl groups,
    as an excipient of a polymeric matrix of a composition providing sustained release of an active ingredient from an oral solid dosage form, which composition further comprising a liquid diluent in a weight ratio of liquid diluent to active ingredient up to 0.85:1, wherein the weight ratio of liquid diluent to solids is of 0.1:1 to 0.85:1.

**12.** The use of claim 11, wherein hydroxy groups of anhydroglucose units of the methylcellulose are substituted with methyl groups such that s23/s26 is 0.12 or more, preferably 0.14 or more, such as 0.16 or more.

**13.** The use of claim 11 or 12, wherein the methylcellulose has a DS(methyl) of from 1.55 to 2.25.

**14.** The use of any one of claims 11 to 13, wherein the solid dosage form is a tablet, pellet or capsule.

**15.** A process for preparing a sustained release composition for oral administration comprising a physiologically active ingredient mixed with a methylcellulose, the process comprising

(a) providing a solution of methylcellulose in a liquid diluent,
(b) mixing the active ingredient in powder or crystalline form and optionally one or more solid excipients with the solution of methylcellulose in a weight ratio of liquid diluent to solids of 0.1:1 to 0.85:1, and
(c) optionally drying the mixture of step (b) to a liquid content of less than 10% by weight of the mixture,
said methylcellulose being composed of anhydroglucose units joined by 1-4 linkages,
wherein hydroxy groups of anhydroglucose units are substituted with methyl groups such that the s23/s26 is 0.27 or less,
wherein s23 is the molar fraction of anhydroglucose units wherein only the two hydroxy groups in the 2- and 3-positions of the anhydroglucose unit are substituted with methyl groups and
wherein s26 is the molar fraction of anhydroglucose units wherein only the two hydroxy groups in the 2- and 6-positions of the anhydroglucose unit are substituted with methyl groups.

**Patentansprüche**

**1.** Zusammensetzung mit anhaltender Wirkstofffreisetzung für die orale Verabreichung, umfassend einen physiologischen Wirkstoff in Abmischung mit einer Methylcellulose, wobei

die Methylcellulose über 1-4-Bindungen verknüpfte Anhydroglucose-Einheiten aufweist und wobei die Hydroxygruppen von Anhydroglucose-Einheiten derart durch Methylgruppen substituiert sind, dass s23/s26 0,27 oder weniger beträgt,
wobei s23 der molare Anteil von Anhydroglucose-Einheiten ist, in denen nur die zwei Hydroxygruppen in der 2- und 3-Position der Anhydroglucose-Einheit durch Methylgruppen substituiert sind, und
wobei s26 der molare Anteil von Anhydroglucose-Einheiten ist, in denen nur die zwei Hydroxygruppen in der 2- und 6-Position der Anhydroglucose-Einheit durch Methylgruppen substituiert sind,
wobei die Zusammensetzung ferner ein flüssiges Verdünnungsmittel in einem Gewichtsverhältnis von flüssigem Verdünnungsmittel zu Wirkstoff bis zu 0,85:1 umfasst, wobei das Gewichtsverhältnis von flüssigem Verdünnungsmittel zu Feststoffen 0,1:1 bis 0,85:1 beträgt.

**2.** Zusammensetzung nach Anspruch 1, wobei das Gewichtsverhältnis von flüssigem Verdünnungsmittel zu Feststoffen bis zu 0,75:1, bevorzugt bis zu 0,70:1, weiter bevorzugt bis zu 0,65:1, bis 0,60:1, bis 0,55:1, bis 0,50:1, bis 0,45:1 oder bis 0,40:1, beträgt.

**3.** Zusammensetzung nach Anspruch 1 oder 2, wobei die Konzentration von Methylcellulose 0,1-10,0 %, bevorzugt 0,2-5,0 %, weiter bevorzugt 0,5-4,0 %, weiter bevorzugt 0,75-2,0 % und noch weiter bevorzugt 0,8-1,5 %, wie 1 %, bezogen auf das Trockengewicht des Wirkstoffs, beträgt.

**4.** Zusammensetzung nach einem der Ansprüche 1 bis 3, umfassend eine Methylcellulose, wobei die Hydroxygruppen von Anhydroglucose-Einheiten derart durch Methylgruppen substituiert sind, dass s23/s26 0,26 oder weniger, bevorzugt 0,25 oder weniger, weiter bevorzugt 0,24 oder weniger, wie 0,22 oder weniger, beträgt.

**5.** Zusammensetzung nach einem der Ansprüche 1 bis 4, ferner umfassend ein Tensid.

**6.** Zusammensetzung nach einem der Ansprüche 1 bis 5, wobei die Konzentration des Tensids im Bereich von 0,1-1,5 Gew.-% der Zusammensetzung liegt.

**7.** Zusammensetzung nach einem der Ansprüche 1 bis 6, ferner umfassend ein Additiv, das mit Magensaft unter Erzeugung eines Gases reagieren kann.

**8.** Zusammensetzung nach Anspruch 7, wobei das Additiv aus Alkalimetall- oder Erdalkalimetallcarbonaten wie $CaCO_3$ oder $Na_2CO_3$ ausgewählt ist.

**9.** Einzeldosisform, umfassend eine Zusammensetzung nach einem der Ansprüche 1-8.

**10.** Einzeldosisform nach Anspruch 9, wobei der Wirkstoff aus der Gruppe bestehend aus Metformin, Metforminhydrochlorid, Acetaminophen und Acetylsalicylsäure ausgewählt ist.

**11.** Verwendung einer Methylcellulose, die aus über 1-4-Bindungen verknüpften Anhydroglucose-Einheiten besteht, wobei die Hydroxygruppen von Anhydroglucose-Einheiten derart durch Methylgruppen substituiert sind, dass s23/s26 0,27 oder weniger beträgt,

wobei s23 der molare Anteil von Anhydroglucose-Einheiten ist, in denen nur die zwei Hydroxygruppen in der 2- und 3-Position der Anhydroglucose-Einheit durch Methylgruppen substituiert sind, und
wobei s26 der molare Anteil von Anhydroglucose-Einheiten ist, in denen nur die zwei Hydroxygruppen in der 2- und 6-Position der Anhydroglucose-Einheit durch Methylgruppen substituiert sind,
als Hilfsstoff einer polymeren Matrix einer Zusammensetzung, die eine anhaltende Freisetzung eines Wirkstoffs aus einer oralen festen Dosisform bereitstellt, wobei die Zusammensetzung ferner ein flüssiges Verdünnungsmittel in einem Gewichtsverhältnis von flüssigem Verdünnungsmittel zu Wirkstoff bis zu 0,85:1 umfasst, wobei das Gewichtsverhältnis von flüssigem Verdünnungsmittel zu Feststoffen 0,1:1 bis 0,85:1 beträgt.

**12.** Verwendung nach Anspruch 11, wobei die Hydroxygruppen von Anhydroglucose-Einheiten der Methylcellulose derart durch Methylgruppen substituiert sind, dass s23/s26 0,12 oder mehr, bevorzugt 0,14 oder mehr, wie 0,16 oder mehr, beträgt.

**13.** Verwendung nach Anspruch 11 oder 12, wobei die Methylcellulose einen DS(Methyl) von 1,55 bis 2,25 aufweist.

**14.** Verwendung nach einem der Ansprüche 11 bis 13, wobei es sich bei der festen Dosisform um eine Tablette, ein Pellet oder eine Kapsel handelt.

**15.** Verfahren zur Herstellung einer Zusammensetzung mit anhaltender Wirkstofffreisetzung für die orale Verabreichung, umfassend einen physiologischen Wirkstoff in Abmischung mit einer Methylcellulose, wobei das Verfahren Folgendes umfasst:

(a) Bereitstellen einer Lösung von Methylcellulose in einem flüssigen Verdünnungsmittel,
(b) Mischen des Wirkstoffs in Pulverform oder kristalliner Form und gegebenenfalls eines oder mehrerer fester Hilfsstoffe mit der Lösung von Methylcellulose in einem Gewichtsverhältnis von flüssigem Verdünnungsmittel zu Feststoffen von 0,01:1 bis 0,85:1 und
(c) gegebenenfalls Trocknen der Mischung aus Schritt (b) bis zu einem Flüssigkeitsgehalt von weniger als 10 Gew.-% der Mischung,
wobei die Methylcellulose aus über 1-4-Bindungen verknüpften Anhydroglucose-Einheiten besteht, wobei die Hydroxygruppen von Anhydroglucose-Einheiten derart durch Methylgruppen substituiert sind, dass s23/s26 0,27 oder weniger beträgt,
wobei s23 der molare Anteil von Anhydroglucose-Einheiten ist, in denen nur die zwei Hydroxygruppen in der 2- und 3-Position der Anhydroglucose-Einheit durch Methylgruppen substituiert sind, und
wobei s26 der molare Anteil von Anhydroglucose-Einheiten ist, in denen nur die zwei Hydroxygruppen in der 2- und 6-Position der Anhydroglucose-Einheit durch Methylgruppen substituiert sind.

## Revendications

**1.** Composition à libération prolongée pour une administration orale comprenant un ingrédient physiologiquement actif mélangé avec une méthylcellulose,

la méthylcellulose ayant des motifs anhydroglucose joints par 1 à 4 liaisons, et des groupes hydroxy de motifs anhydroglucose étant substitués par des groupes méthyle de sorte que le s23/s26 est de 0,27 ou moins,
s23 étant la fraction molaire de motifs anhydroglucose dans lesquels seulement les deux groupes hydroxy dans les positions 2 et 3 du motif anhydroglucose sont substitués par des groupes méthyle et

s26 étant la fraction molaire de motifs anhydroglucose dans lesquels seulement les deux groupes hydroxy dans les positions 2 et 6 du motif anhydroglucose sont substitués par des groupes méthyle,
la composition comprenant en outre un diluant liquide en un rapport en poids de diluant liquide sur ingrédient actif allant jusqu'à 0,85 : 1, le rapport en poids de diluant liquide sur solides étant de 0,1 : 1 à 0,85 : 1.

**2.** Composition selon la revendication 1, le rapport en poids de diluant liquide sur ingrédient actif allant jusqu'à 0,75 : 1, préférablement jusqu'à 0,70 : 1, plus préférablement jusqu'à 0,65 : 1, jusqu'à 0,60 : 1, jusqu'à 0,55 : 1, jusqu'à 0,50 : 1, jusqu'à 0,45 : 1 ou jusqu'à 0,40 : 1.

**3.** Composition selon la revendication 1 ou 2, la concentration de méthylcellulose étant de 0,1 à 10,0 %, préférablement 0,2 à 5,0 %, plus préférablement 0,5 à 4,0 %, plus préférablement 0,75 à 2,0 % et encore plus préférablement 0,8 à 1,5 %, tel que 1 %, en poids sec de l'ingrédient actif.

**4.** Composition selon l'une quelconque des revendications 1 à 3 comprenant une méthylcellulose dans laquelle des groupes hydroxy de motifs anhydroglucose sont substitués par des groupes méthyle de sorte que s23/s26 est de 0,26 ou moins, préférablement 0,25 ou moins, plus préférablement 0,24 ou moins, tel que 0,22 ou moins.

**5.** Composition selon l'une quelconque des revendications 1 à 4 comprenant en outre un tensioactif.

**6.** Composition selon l'une quelconque des revendications 1 à 5, la concentration du tensioactif étant dans la plage de 0,1 à 1,5 % en poids de la composition.

**7.** Composition selon l'une quelconque des revendications 1 à 6 comprenant en outre un additif capable de réagir avec un fluide gastrique pour générer un gaz.

**8.** Composition selon la revendication 7, l'additif étant choisi parmi des carbonates de métaux alcalins ou de métaux alcalino-terreux tels que $CaCO_3$ ou $Na_2CO_3$.

**9.** Forme posologique unitaire comprenant une composition selon l'une quelconque des revendications 1 à 8.

**10.** Forme posologique unitaire selon la revendication 9, l'ingrédient actif étant choisi dans le groupe constitué par la metformine, le chlorhydrate de metformine, l'acétaminophène et l'acide acétylsalicylique.

**11.** Utilisation d'une méthylcellulose composée de motifs anhydroglucose joints par 1 à 4 liaisons, des groupes hydroxy de motifs anhydroglucose étant substitués par des groupes méthyle de sorte que le s23/s26 est de 0,27 ou moins,

s23 étant la fraction molaire de motifs anhydroglucose dans lesquels seulement les deux groupes hydroxy dans les positions 2 et 3 du motif anhydroglucose sont substitués par des groupes méthyle et
s26 étant la fraction molaire de motifs anhydroglucose dans lesquels seulement les deux groupes hydroxy dans les positions 2 et 6 du motif anhydroglucose sont substitués par des groupes méthyle,
en tant qu'un excipient d'une matrice polymérique d'une composition fournissant une libération prolongée d'un ingrédient actif d'une forme posologique solide orale, laquelle composition comprend en outre un diluant liquide en un rapport en poids de diluant liquide sur ingrédient actif allant jusqu'à 0,85 : 1, le rapport en poids de diluant liquide sur solides étant de 0,1 : 1 à 0,85 : 1.

**12.** Utilisation selon la revendication 11, des groupes hydroxy de motifs anhydroglucose de la méthylcellulose étant substitués par des groupes méthyle de sorte que s23/s26 est de 0,12 ou plus, préférablement 0,14 ou plus, tel que 0,16 ou plus.

**13.** Utilisation selon la revendication 11 ou 12, la méthylcellulose ayant un DS(méthyle) allant de 1,55 à 2,25.

**14.** Utilisation selon l'une quelconque des revendications 11 à 13, la forme posologique solide étant un comprimé, une pastille ou une capsule.

**15.** Procédé pour la préparation d'une composition à libération prolongée pour une administration orale comprenant un ingrédient physiologiquement actif mélangé avec une méthylcellulose, le procédé comprenant

(a) la fourniture d'une solution de méthylcellulose dans un diluant liquide,

(b) le mélange de l'ingrédient actif sous forme de poudre ou cristalline et éventuellement d'un ou plusieurs excipients solides avec la solution de méthylcellulose en un rapport en poids de diluant liquide sur solides de 0,1 : 1 à 0,85 : 1, et
(c) éventuellement le séchage du mélange de l'étape (b) jusqu'à une teneur en liquide de moins de 10 % en poids du mélange,
ladite méthylcellulose étant composée de motifs anhydroglucose joints par 1 à 4 liaisons, des groupes hydroxy de motifs anhydroglucose étant substitués par des groupes méthyle de sorte que le s23/s26 est de 0,27 ou moins, s23 étant la fraction molaire de motifs anhydroglucose dans lesquels seulement les deux groupes hydroxy dans les positions 2 et 3 du motif anhydroglucose sont substitués par des groupes méthyle et
s26 étant la fraction molaire de motifs anhydroglucose dans lesquels seulement les deux groupes hydroxy dans les positions 2 et 6 du motif anhydroglucose sont substitués par des groupes méthyle.

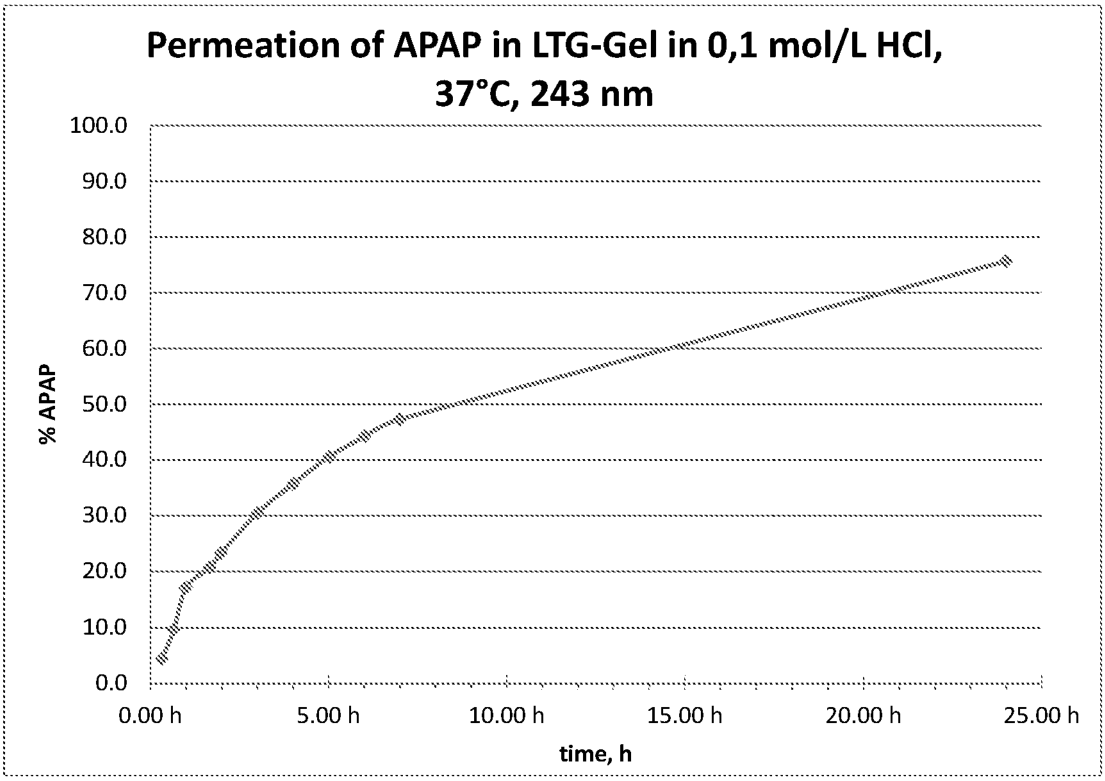
Permeation of APAP in LTG-Gel in 0,1 mol/L HCl, 37°C, 243 nm
% APAP
100.0
90.0
80.0
70.0
60.0
50.0
40.0
30.0
20.0
10.0
0.0
0.00 h
5.00 h
10.00 h
15.00 h
20.00 h
25.00 h
time, h

Fig. 1

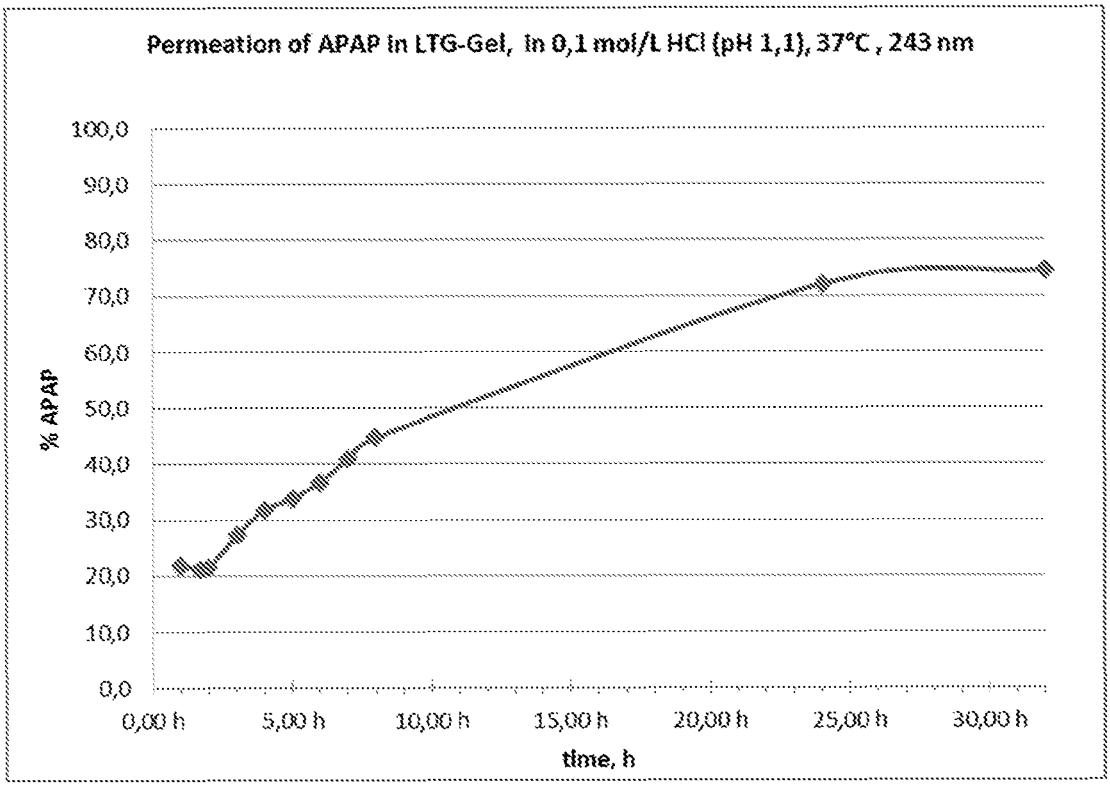
Permeation of APAP in LTG-Gel, in 0,1 mol/L HCl (pH 1,1), 37°C, 243 nm
100,0
90,0
80,0
70,0
60,0
50,0
40,0
30,0
20,0
10,0
0,0
% APAP
0,00 h
5,00 h
10,00 h
15,00 h
20,00 h
25,00 h
30,00 h
time, h

Fig. 2

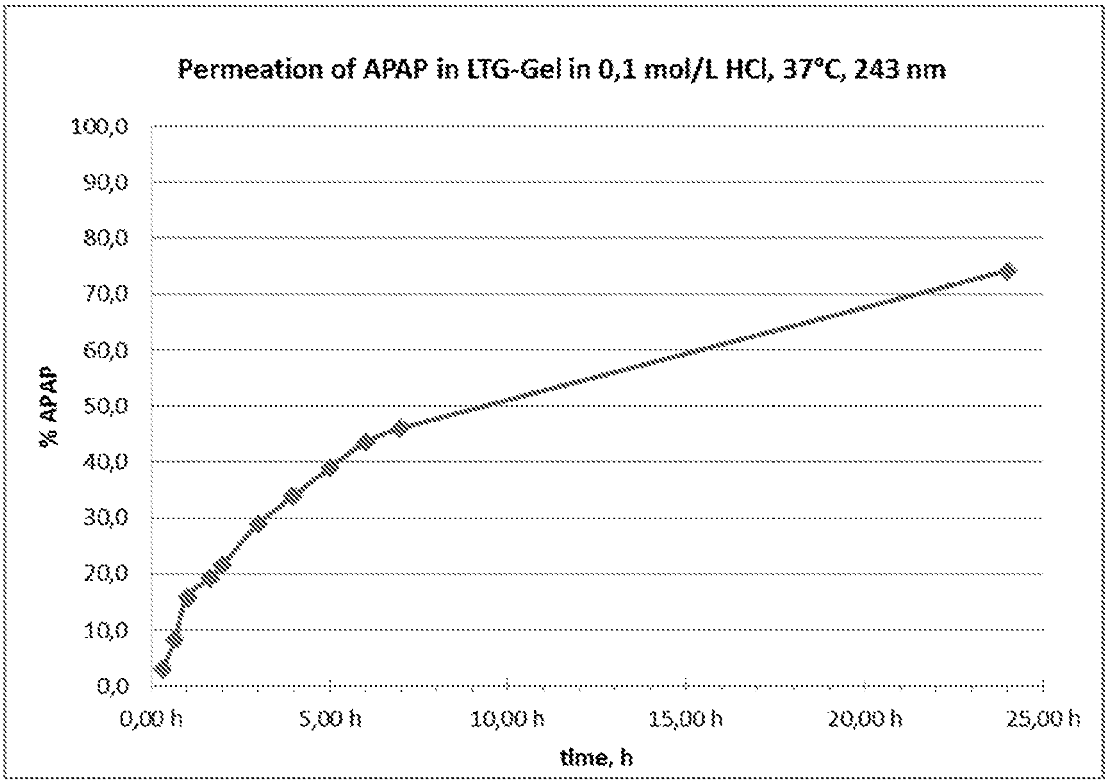
Permeation of APAP in LTG-Gel in 0,1 mol/L HCl, 37°C, 243 nm
100,0
90,0
80,0
70,0
60,0
50,0
40,0
30,0
20,0
10,0
0,0
% APAP
0,00 h
5,00 h
10,00 h
15,00 h
20,00 h
25,00 h
time, h

Fig. 3

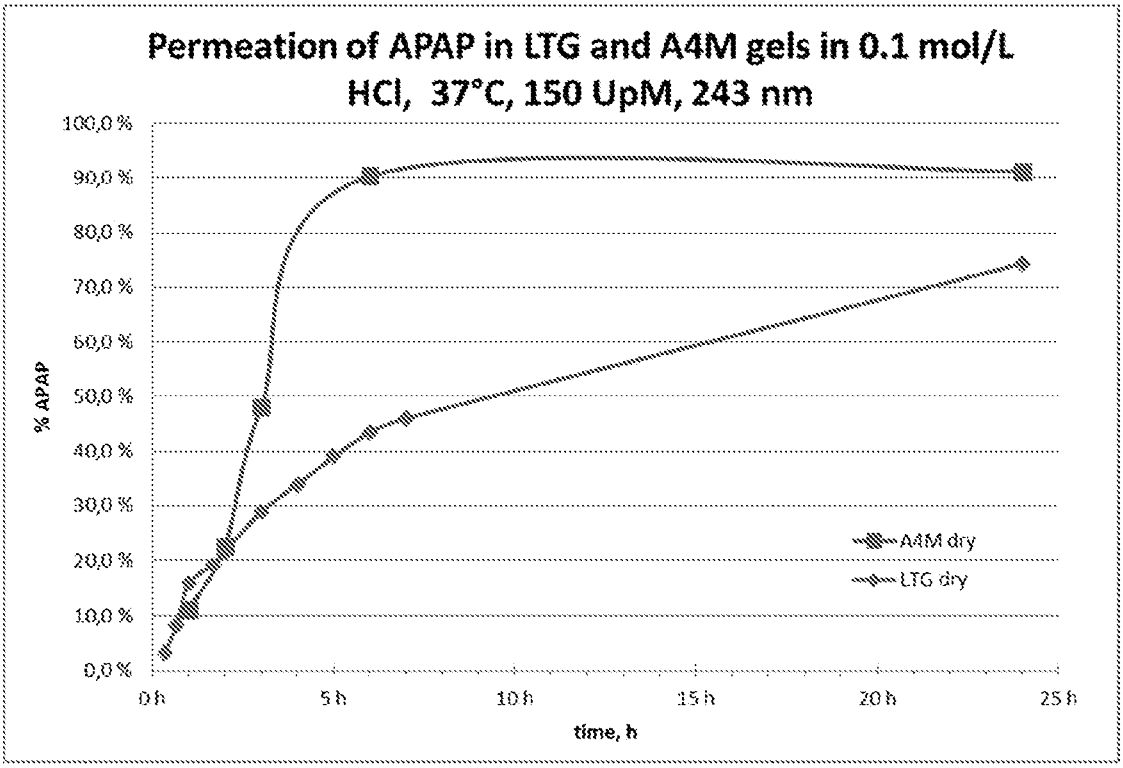
Permeation of APAP in LTG and A4M gels in 0.1 mol/L HCl, 37°C, 150 UpM, 243 nm
100,0 %
90,0 %
80,0 %
70,0 %
60,0 %
50,0 %
40,0 %
30,0 %
20,0 %
10,0 %
0,0 %
% APAP
0 h
5 h
10 h
15 h
20 h
25 h
time, h
A4M dry
LTG dry

Fig. 4

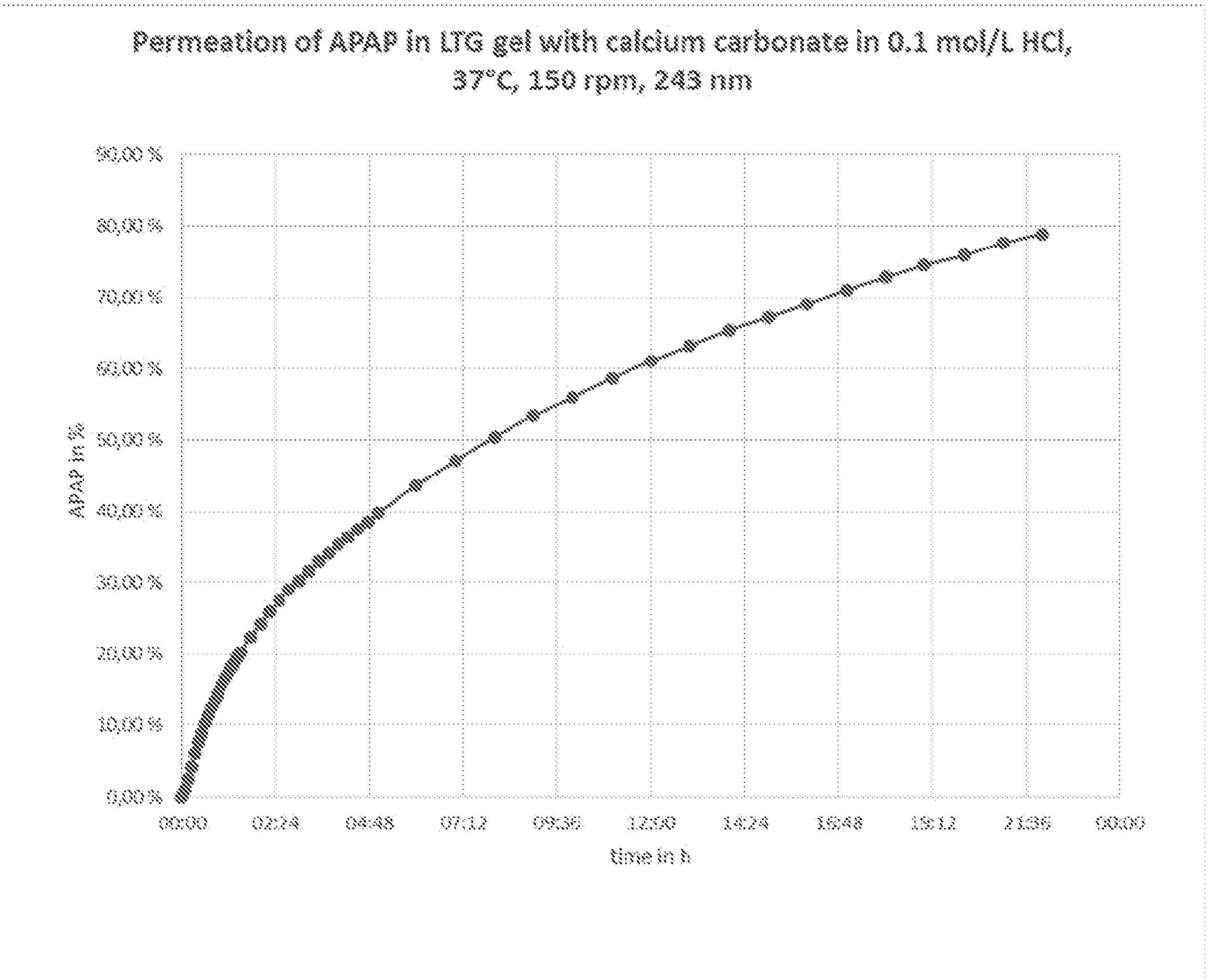
Permeation of APAP in LTG gel with calcium carbonate in 0.1 mol/L HCl,
37°C, 150 rpm, 243 nm
90,00 %
80,00 %
70,00 %
60,00 %
50,00 %
40,00 %
30,00 %
20,00 %
10,00 %
0,00 %
APAP in %
00:00
02:24
04:48
07:12
09:36
12:00
14:24
16:48
19:12
21:36
00:00
time in h

Fig. 5

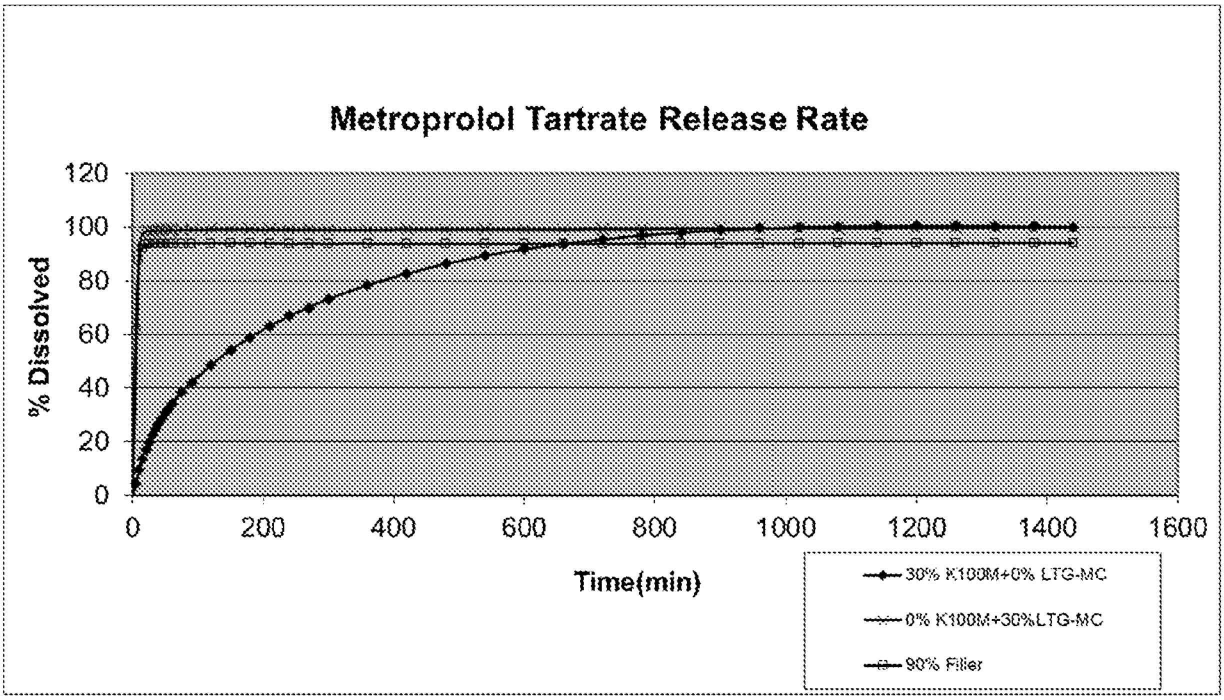
Metroprolol Tartrate Release Rate
% Dissolved
120
100
80
60
40
20
0
0
200
400
600
800
1000
1200
1400
1600
Time(min)
30% K100M+0% LTG-MC
0% K100M+30%LTG-MC
90% Filier

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4734285 A
- WO 2015009796 A
- EP 2997098 B1
- US 20170260414 A
- US 9700630 B2
- EP 1141029 A
- EP 210917 A
- EP 1423433 A
- US 4316982 A


### Non-patent literature cited in the description


- **MITCHELL K et al.** *International Journal of Pharmaceutics,* 1993, vol. 100 (1-3), 143-154
- **K.S. AITHAL et al.** *Indian J. Dent. Res. 1,* April 1990, 174-181
- Ethyl Hydroxyethyl Cellulose in Carbohydrate Research. Elsevier Science Publishers B.V, 1988, vol. 176, 137-144
- **R.G. ACKMAN.** *J. Gas Chromatogr,* 1964, vol. 2, 173-179
- **R.F. ADDISON ; R.G. ACKMAN.** *J. Gas Chromatogr.,* 1968, vol. 6, 135-138
- **D.P. SWEET ; R.H. SHAPIRO ; P. ALBERSHEIM.** *Carbohyd. Res,* 1975, vol. 40, 217-225